(19) Europäisches Patentamt
European Patent Office
Office européen des brevets

(11) **EP 3 147 318 B1**

(12) **EUROPÄISCHE PATENTSCHRIFT**

(45) Veröffentlichungstag und Bekanntmachung des Hinweises auf die Patenterteilung:
**11.03.2020 Patentblatt 2020/11**

(51) Int Cl.:
*C08K 5/00* (2006.01)          *C08K 5/12* (2006.01)
*C08L 27/06* (2006.01)

(21) Anmeldenummer: **16185659.6**

(22) Anmeldetag: **25.08.2016**

(54) **TRIPENTYLESTER DER TRIMELLITSÄURE**

TRIPENTYLESTER OF TRIMELLITIC ACID

ESTER DE TRIPHÉNYLE DE L'ACIDE TRIMÉLLITIQUE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **28.09.2015 EP 15187128**

(43) Veröffentlichungstag der Anmeldung:
**29.03.2017 Patentblatt 2017/13**

(60) Teilanmeldung:
**17189749.9 / 3 287 484**

(73) Patentinhaber: **Evonik Operations GmbH 45128 Essen (DE)**

(72) Erfinder:
• **Woldt, Benjamin**
  **44892 Bochum (DE)**
• **Grass, Michael**
  **45721 Haltern am See (DE)**
• **Boeck, Florian**
  **48143 Münster (DE)**

(74) Vertreter: **Evonik Patent Association c/o Evonik Industries AG IP Management Bau 1042A/PB 15 Paul-Baumann-Straße 1 45772 Marl (DE)**

(56) Entgegenhaltungen:
**EP-A2- 0 157 583      FR-A1- 2 102 272 US-A- 6 156 239**

• **DRECHSLER, GERHARD ET AL: "Trimellitic and isophthalic acids and their esters", JOURNAL FUER PRAKTISCHE CHEMIE (LEIPZIG) , 27(5-6), 231-8 CODEN: JPCEAO; ISSN: 0021-8383, 1965, XP009187476,**

Bemerkungen:
Die Akte enthält technische Angaben, die nach dem Eingang der Anmeldung eingereicht wurden und die nicht in dieser Patentschrift enthalten sind.

**Beschreibung**

Die vorliegende Erfindung betrifft Tripentylester der Trimellitsäure.

Polyvinylchlorid (PVC) gehört zu den wirtschaftlich bedeutendsten Polymeren. Es findet sowohl als Hart-PVC als auch als Weich-PVC vielfältige Anwendungen.

Zur Erzeugung von PVC-basierten Produkten werden dem PVC zur Verbesserung der Verarbeitbarkeit sowie zur Anpassung weiterer anwendungsrelevanter Eigenschaften Weichmacher zugesetzt. Aufgrund ihrer vorteilhaften Eigenschaften gehören zu den wichtigsten Weichmachern für PVC und Vinylchlorid-haltige Copolymere noch immer Verbindungen aus der Gruppe der Phthalate, insbesondere Diethylhexylphthalat (DEHP), Diisononylphthalat (DINP) und Diisodecylphthalat (DIDP). Auf Grund von Diskussionen über eventuelle toxikologische Effekte dieser Stoffklasse, wird seit vielen Jahren nach Alternativen für Phthalat-basierte Weichmacher gesucht.

Als neue Weichmacherklasse werden in dem Artikel "Vinyl Plasticizers form Trimellitic Anhydride" (Soc. Plastics Engrs. Techn. Papers. Volume 8, Issue Session 22, Pages Paper 1,1-9, Journal 1962) Triester der Trimellitsäure (Trimellitate) vorgestellt und die Eigenschaften der Methyl-, Ethyl-, Butyl-, *n*-Hexyl-, *iso*-Octyl-, 2-Ethylhexyl-, *n*-Octyl-, *iso*-Decyl-, *n*-Octyldecyl- und Dibutylbenzyl-Triester der Trimellitsäure den Eigenschaften anderer Weichmacher wie Dioctylphthalat und Diisooctylphthalat gegenübergestellt. Die in diesem Artikel vorgestellten Untersuchungen zeigen, dass Tributyltrimellitat eine gute Verarbeitbarkeit aufweist, die Verarbeitbarkeit der Trimellitate jedoch mit zunehmender Länge des Alkylrestes abnimmt.

Das Patent EP 354 700 B1 beschreibt die Eignung von Gemischen von Trimellitaten, welche Alkylreste mit unterschiedlicher Kohlenstoffanzahl aufweisen, wobei mindestens 85 Mol-% der Alkylreste linear sind, zum Pulverformen einer PVC-Zusammensetzung, beschreibt jedoch auch, dass einige Trimellitate schlecht von PVC-Partikeln adsorbiert werden, also eine schlechte Verarbeitbarkeit aufweisen.

Eine gute Verträglichkeit von gemischten Trimellitaten des Phenylpropanols und eines Alkohols mit 4 bis 10 Kohlenstoffatomen mit PVC und Vinylchlorid-haltigen Copolymeren offenbart die Patentschrift DE 23 48 511 C3. Der Rohstoff Phenylpropanol ist jedoch teuer in der Herstellung und wird zudem als haut- und augenreizend eingestuft.

Die Offenlegungsschrift WO 91/07459 A1 empfiehlt den Einsatz von Trimellitaten, insbesondere von Tridecyltrimellitat, Tridodecyltrimellitat, Tri-*n*-octyltrimellitat und gemischten $C_{8-10}$-Trimellitaten als Weichmacher für Styrol-Acrylonitril-Copolymere und empfiehlt ausdrücklich lineare Alkylreste.

In dem Patent EP 479 260 B1 werden Cellulosefilme beschrieben, welche neben einem Phosphorsäureesterweichmacher einen Trimellitat-Weichmacher, insbesondere Trimethyl- oder Triethyltrimellitat enthalten.

Das Fachbuch "Plasticizers - Principles and Practice" von A.S. Wilson (The Institute of Materials, 1995, Seiten 166 bis 170) schreibt den Trimellitaten gute Migrationseigenschaften zu, klassifiziert jedoch ausschließlich Trimellitate mit Alkoholresten enthaltend 7 bis 9 Kohlenstoffatome oder Gemische aus $C_6$- und $C_8$-Estern oder $C_7$-, $C_8$- und $C_9$-Estern der Trimellitsäure als kommerziell interessant, wobei Tri-2-ethylhexyltrimellitat als das wichtigste Trimellitat herausgestellt wird.

Die Offenlegungsschrift JP 2005/230058 A schlägt vor, die für Medizinanwendungen nicht ausreichenden Migrationseigenschaften von Tri-2-ethylhexyltrimellitat durch die Zumischung von epoxidierten Pflanzenölen zu verbessern.

Drechsler, Gerhard et al. "Trimellic and isophthalic acids and their esters", Journal für praktische Chemie, 4. Reihe, Band 27, Seiten 231 - 238 offenbart einzelne Trimellitate, darunter die Isoamylester (Methylbutylester) und lineare Pentylester und deren Eignung als Weichmacher.

Der vorliegenden Erfindung lag nun die Aufgabe zugrunde, Weichmacher bereitzustellen, die als Phthalat-Ersatz einsetzbar sind. Diese Weichmacher sollten eine Verarbeitbarkeit mit Polymeren, insbesondere mit PVC und Vinylchlorid-haltigen Copolymeren aufweisen, welche zumindest auf dem Niveau von DINP und Tri-2-ethylhexyltrimellitat liegt, vorzugsweise noch besser ist. Bevorzugt sollten diese Weichmacher eine geringe Migrationsneigung aus weichgemachtem PVC oder weichgemachten Vinylchlorid-haltigen Copolymeren in andere Materialien aufweisen.

Diese Aufgabe wird gelöst durch die Verwendung von Gemischen gemäß Anspruch 1.

Gegenstand der vorliegenden Erfindung ist die Verwendung von Gemischen von Tri*iso*pentylestern der Trimellitsäure umfassend isomere Pentylreste, in welchen mindestens 20 Mol-% der im Estergemisch gebundenen isomeren Pentylreste verzweigt sind und gleichzeitig mehr als 10 Mol-% der im Estergemisch gebundenen isomeren Pentylreste linear sind, als Weichmacher oder Teil einer Weichmacherzusammensetzung, welche neben den Gemischen der Tri*iso*pentylester der Trimellitsäure mindestens eine weitere Polymer-weichmachende Verbindung enthält, für PVC oder Vinylchlorid-haltige Polymere.

Die Silbe *"iso"* kennzeichnet, dass es sich um ein Gemisch mehrerer Isomere mit gemeinsamer Kohlenstoffanzahl, hier mit 5 Kohlenstoffatomen, handelt. Übereinstimmend mit der geläufigen Bedeutung des Begriffes Gemisch, umfassen erfindungsgemäße Gemische stets mindestens zwei unterschiedliche Isomere des Tripentylesters der Trimellitsäure.

Entsprechende Gemische werden im Folgenden auch als erfindungsgemäße Gemische oder erfindungsgemäße Gemische von Tri*iso*pentylestern (der Trimellitsäure) bezeichnet.

Überraschenderweise wurde gefunden, dass erfindungsgemäße Gemische eine bessere Verarbeitbarkeit mit PVC aufweisen als DINP und Tri-2-ethylhexyltrimellitat und gleichzeitig eine sehr geringere Migrationsneigung in nicht weichgemachtes PVC (rPVC) oder in hochschlagfestes Polystyrol (HIPS) aufweisen, welche geringer ist als die von DINP, Tri-*n*-butyltrimellitat, Tri-*n*-pentyltrimellitat und Tri-*n*-hexyltrimellitat. Zudem weisen Plastisole oder Halbzeuge, welche erfindungsgemäße Gemische enthalten, eine geringere Flüchtigkeit auf als vergleichbare Plastisole oder Halbzeuge enthaltend DINP oder Tri-*n*-butyltrimellitat. Darüber hinaus können die erfindungsgemäßen Gemische bei deutlich niedrigeren Temperaturen beziehungsweise bei gleicher Temperatur wesentlich schneller verarbeitet werden als Trimellitate, welche sechs und mehr Kohlenstoffatome im Alkylrest enthalten. Somit weisen die erfindungsgemäßen Gemische eine einzigartige Kombination von guter Verarbeitbarkeit, geringer Migrationsneigung und geringer Flüchtigkeit auf.

Bevorzugt sind erfindungsgemäße Gemische, in welchen mindestens 25 Mol-%, besonders bevorzugt mindestens 50 Mol-%, weiter bevorzugt mindestens 67 Mol-%, ganz besonders bevorzugt mindestens 75 Mol-% und insbesondere mindestens 80 Mol-% der im Estergemisch gebundenen isomeren Pentylreste verzweigt sind, da gezeigt werden konnte, dass solche Gemische eine besonders niedrige Migrationsneigung in andere Polymere zeigen.

Ein verzweigter Pentylrest ist vorzugsweise ein Methylbutyl-Rest. Dementsprechend sind erfindungsgemäße Gemische bevorzugt, in welchen die verzweigten Pentylreste zu mindestens 50 Mol-%, bevorzugt zu mindestens 60 Mol-%, weiter bevorzugt zu mindestens 70 Mol-%, bevorzugt dazu zu mindestens 80 Mol-%, ganz besonders bevorzugt zu mindestens 90 Mol-% und insbesondere zu mindestens 95 Mol-% aus Methylbutylresten bestehen.

Ebenfalls gefunden wurde, dass die Migrationsneigung besonders niedrig ist, wenn die verzweigten isomeren Pentylreste einen großen Anteil an 2-Methylbutylresten aufweisen. In einer bevorzugten Ausführungsform sind deshalb mindestens 50 Mol-%, vorzugsweise mindestens 60 Mol-%, bevorzugt mindestens 70 Mol-%, weiter bevorzugt mindestens 80 Mol-%, besonders bevorzugt mindestens 90 Mol-% und insbesondere mindestens 95 Mol-% der im Estergemisch gebundenen verzweigten isomeren Pentylreste 2-Methylbutylreste.

In einer besonders bevorzugten Ausführungsform besteht das erfindungsgemäße Gemisch zu mindestens 75 Mol-% und insbesondere zu mindestens 90 Mol-% aus Estern, welche 2-Methylbutyl- und/oder lineare Pentylreste enthalten, wobei das Molverhältnis von 2-Methylbutyl-Resten zu linearen Pentylresten innerhalb dieser Ester im Bereich von 5:95 bis 80:20, bevorzugt im Bereich von 10:90 bis 75:25 und insbesondere im Bereich von 20:80 oder 30:70 bis 70:30 liegt.

Es kann aber auch von Vorteil sein, insbesondere wenn die maximale Viskosität während der Gelierung eines ein erfindungsgemäßes Gemisch enthaltenden Plastisols besonders niedrig gehalten werden soll, wenn die verzweigten isomeren Pentylreste zu einem großen Anteil aus 3-Methylbutylresten bestehen. In einem solchen Fall sind mindestens 10 Mol-%, vorzugsweise mindestens 20 Mol-%, bevorzugt mindestens 30 Mol-%, weiter bevorzugt mindestens 40 Mol-%, mit Vorzug dazu mindestens 50 Mol-%, vorzugsweise mindestens 60 Mol-%, weiter bevorzugt mindestens 70 Mol-%, besonders bevorzugt mindestens 80 Mol-% und insbesondere mindestens 90 Mol-% der im Estergemisch gebundenen verzweigten isomeren Pentylreste 3-Methylbutylreste. Es kann des Weiteren von Vorteil sein, wenn das erfindungsgemäße Gemisch zu mindestens 75 Mol-% und insbesondere zu mindestens 90 Mol-% aus Estern besteht, welche 3-Methylbutyl- und/oder linearen Pentylreste enthalten, und dabei das Molverhältnis von 3-Methylbutyl-Resten zu linearen Pentylresten bei 5:95 bis 80:20, bevorzugt im Bereich von 10:90 bis 75:25 und insbesondere im Bereich von 20:80 oder 30:70 bis 70:30 liegt.

Weiter wurde gefunden, dass die Verarbeitbarkeit der erfindungsgemäßen Gemische sowie die Effizienz der erfindungsgemäßen Gemische mit zunehmenden Gehalt an linearen Pentylresten steigen. Aus diesem Grunde kann es von Vorteil sein, wenn mehr als 20 Mol-%, weiter bevorzugt mehr als 30 Mol-%, besonders bevorzugt mehr als 40 Mol-%, ganz besonders bevorzugt mehr als 50 Mol-%, mit Vorzug dazu mehr als 60 Mol-%, vorzugsweise mehr als 70 Mol-% und insbesondere mehr als 80 Mol-% der im Estergemisch gebundenen isomeren Pentylreste linear sind. Es kann aufgrund einer verringerten Migrationsneigung auch bevorzugt sein, dass der Anteil der linearen Pentylreste an den im Estergemisch gebundenen isomeren Pentylresten bei weniger als 50 Mol-% und bevorzugt bei weniger als 30 Mol-% liegt.

Vorzugsweise basieren die im Gemisch der Tri*iso*pentylester der Trimellitsäure gebundenen isomeren Pentylreste auf Gemischen primärer Pentanole, da diese eine höhere Effizienz und eine bessere Verarbeitbarkeit aufweisen als die entsprechenden Tri*iso*pentylester, welche auf sekundären Alkoholen basieren.

Ein bevorzugter Gegenstand der vorliegenden Erfindung ist die Verwendung eines Gemisches von Tri*iso*pentylestern der Trimellitsäure umfassend isomere Pentylreste, in welchen mindestens 20 Mol-% der im Estergemisch gebundenen isomeren Pentylreste verzweigt sind und dabei vorzugsweise mindestens 50 Mol-% und insbesondere mindestens 90 Mol-% der im Estergemisch gebundenen verzweigten isomeren Pentylreste 2-Methylbutylreste sind.

Bevorzugt enthält dieses Gemisch mindestens 67 Mol-% und insbesondere mindestens 80 Mol-% verzweigte Pentylreste basierend auf allen im Estergemisch gebundenen isomeren Pentylresten.

Ein weiterer bevorzugter Gegenstand der vorliegenden Erfindung ist die Verwendung eines Gemisches von Tri*iso*pentylestern der Trimellitsäure umfassend isomere Pentylreste, in welchem mindestens 20 Mol-% der im Estergemisch gebundenen isomeren Pentylreste verzweigt und mehr als 20 Mol-%, vorzugsweise mehr als 50 Mol-% und insbesondere mehr als 85 Mol-% der im Estergemisch gebundenen isomeren Pentylreste linear sind. Vorzugsweise sind dabei mindestens 50 Mol-%, vorzugsweise mindestens 80 Mol-% und insbesondere mindestens 95 Mol-% der im Estergemisch gebundenen verzweigten isomeren Pentylreste 2-Methylbutylreste.

Ein Gegenstand der vorliegenden Erfindung ist zudem die Verwendung von Gemischen von Tri*iso*pentylestern der Trimellitsäure umfassend isomere Pentylreste, bei welcher die Gemische von Tri*iso*pentylestern der Trimellitsäure hergestellt werden, indem Trimellitsäure und/oder ein Trimellitsäurederivat mit einem Gemisch isomerer Pentanole umgesetzt wird/werden.

Geeignete Trimellitsäurederivate sind Trimellitsäureanhydrid, Säurehalogenide der Trimellitsäure und Trimellitsäuretriester.

Mit Vorzug sind in diesem Herstellungsverfahren mindestens 20 Mol-%, bevorzugt mindestens 25 Mol-%, besonders bevorzugt mindestens 50 Mol-%, weiter bevorzugt mindestens 67 Mol-%, ganz besonders bevorzugt mindestens 75 Mol-% und insbesondere mindestens 80 Mol-% der im Gemisch der isomeren Pentanole gebundenen isomeren Pentylreste verzweigt, wobei die verzweigten Pentylreste vorzugsweise zu mindestens 80 Mol-% aus Methylbutylresten bestehen.

Besonders vorteilhafte Gemische werden erhalten, wenn in dem Verfahren mindestens 50 Mol-%, vorzugsweise mindestens 60 Mol-%, bevorzugt mindestens 70 Mol-%, weiter bevorzugt mindestens 80 Mol-%, besonders bevorzugt mindestens 90 Mol-% und insbesondere mindestens 95 Mol-% der im Gemisch der isomeren Pentanole gebundenen verzweigten isomeren Pentylreste 2-Methylbutylreste sind.

In einigen Ausführungsformen ist es bevorzugt, wenn in dem Verfahren ein Gemisch isomerer Pentanole eingesetzt wird, in welchem mehr als 20 Mol-%, weiter bevorzugt mehr als 30 Mol-%, besonders bevorzugt mehr als 40 Mol-%, ganz besonders bevorzugt mehr als 50 Mol-%, mit Vorzug dazu mehr als 60 Mol-%, vorzugsweise mehr als 70 Mol-% und insbesondere mehr als 80-% der im Gemisch der isomeren Pentanole gebundenen isomeren Pentylreste linear sind.

Bevorzugt wird zur Herstellung der erfindungsgemäßen Gemische von Tri*iso*pentylestern der Trimellitsäure das Trimellitsäurederivat Trimellitsäureanhydrid eingesetzt.

Werden die erfindungsgemäßen Gemische durch Umesterung hergestellt, so wird/werden vorzugsweise ein oder mehrere Trimellitsäuretrialkylester, in dem/denen die Alkylreste der Esterfunktionen jeweils weniger als 4 Kohlenstoffatome umfassen, mit einem Gemisch isomerer Pentanole umgeestert, wobei mehr als 5 Mol-% der im Gemisch der isomeren Pentanole gebundenen isomeren Pentylreste verzweigt sind.

Bevorzugt wird Trimellitsäuretrimethylester oder Trimellitsäuretriethylester, insbesondere Trimellitsäuretrimethylester zu den erfindungsgemäßen Gemischen von Tri*iso*pentylestern der Trimellitsäure umgeestert.

Die Veresterung beziehungsweise Umesterung wird vorzugsweise in Anwesenheit eines Katalysators oder mehrerer Katalysatoren, beispielsweise unter Verwendung von Brönstedt- oder Lewis-Säuren oder -Basen als Katalysator, durchgeführt. Als besonders geeignete Katalysatoren haben sich Schwefelsäure, Methansulfonsäure, *p*-Toluolsulfonsäure und Metallverbindungen erwiesen. Beispiele für besonders bevorzugte Katalysatoren sind Zinnpulver, Zinn(II)oxid, Zinn(II)oxalat, Titankatalysatoren wie Tetra*iso*propylorthotitanat, Tetrabutylorthotitanat oder Tetrapentylorthotitanat sowie Zirkoniumester wie Tetrabutylzirkonat oder Tetrapentylzirkonat. Beispiele für besonders bevorzugte basische Katalysatoren sind Alkoholate wie Natriummethanolat und Kaliummethanolat.

Um das sich bei der Reaktion ausbildende Gleichgewicht zu Gunsten der erfindungsgemäßen Gemische zu verschieben, kann es vorteilhaft sein, das bei der Veresterung entstehende Wasser beziehungsweise den bei der Umesterung entstehenden Alkohol aus dem Reaktionsgemisch abzudestillieren. Vorzugsweise wird ein Azeotrop von Wasser und Alkohol abdestilliert. Aufgrund einer möglichen Schaumbildung kann hier mit einer Kolonne gearbeitet werden.

Zudem kann es vorteilhaft sein, das Gemisch isomerer Pentanole insgesamt im Überschuss einzusetzen. Vorzugsweise wird das Gemisch isomerer Pentanole in einem Überschuss von 5 bis 50 Mol-%, insbesondere 9 bis 30 Mol-% der zur Bildung des erfindungsgemäßen Gemisches von Tri*iso*pentylestern der Trimellitsäure notwendigen molaren Menge eingesetzt. Mit Vorzug wird der nach Beendigung der Reaktion übrig bleibende Überschussalkohol für eine weitere Veresterung oder Umesterung oder eine andere chemische Reaktion wiederverwendet. Hierzu kann der Überschussalkohol zur Erhöhung seiner Reinheit aufgearbeitet werden. So ist es beispielsweise möglich, ein abdestilliertes Alkohol-Wasser-Azeotrop zumindest teilweise zu kondensieren, das Kondensat in eine wässrige und eine organische Phase aufzutrennen, aus der organischen Phase unerwünschte Nebenprodukte - wie beispielsweise durch Wasserabspaltung aus dem Alkohol gebildete Olefine - abzutrennen, bevor die dann aufgereinigte organische Phase wieder in das Reaktionssystem zurückführt wird oder für eine andere Reaktion oder zu einem anderen Zweck Verwendung findet.

Möglich ist es zudem, das Reaktionsgemisch der Veresterung beziehungsweise Umesterung mit überhitztem Alkoholdampf zu behandeln. Hierdurch kann ein Teil des Energieeintrages durch andere Medien eingespart sowie eine gute Durchmischung des Reaktionsmediums erreicht werden.

Andere Möglichkeiten der Energieeinsparung bestehen darin, das Gemisch isomerer Pentanole mit einer Temperatur oberhalb der Umgebungstemperatur, beispielsweise mit 40 °C oder 60°C in das Reaktionssystem einzuspeisen. Auch Trimellitsäureanhydrid kann mit erhöhter Temperatur, vorzugsweise als Schmelze in dem erfindungsgemäßen Verfahren eingesetzt werden. Neben dem Vorteil des Energieeintrages ermöglicht diese Verfahrensweise zudem eine bessere Durchmischung des Reaktionsmediums und eine rascher ablaufende Reaktion.

Bei der Herstellung erfindungsgemäßer Gemische von Tri*iso*pentylestern der Trimellitsäure aus Trimellitsäure, Trimellitsäureanhydrid oder Trimellitsäuretrialkylester, insbesondere aus Trimellitsäureanhydrid, wird vorzugsweise das Reaktionsgemisch zum Sieden erhitzt und es werden mit Vorzug mindestens 0,2 Mol-Äquivalent, vorzugsweise mindestens 0,25 Mol-Äquivalent und insbesondere mindestens 0,3 Mol-Äquivalent der Menge an Gemisch isomerer Pentanole, welche zur Einführung aller im Estergemisch gebundenen isomeren Pentylreste notwendig ist, dem die Trimellitsäure und/oder das Trimellitsäurederivat enthaltenden Reaktionsgemisch erst nach Erreichen der Siedetemperatur hinzugegeben. Bei der Berechnung der Menge des Gemisches isomerer Pentanole, welche erst nach Erreichen der Siedetemperatur dem Reaktionsgemisch hinzugegeben wird, ist zu berücksichtigen, dass auch etwaig eingesetzte Überschussmengen an isomeren Pentanolen erst nach Erreichen der Siedetemperatur dem Reaktionsgemisch zugesetzt werden. Eine solche Reaktionsführung ermöglicht es, höhere Ausbeuten an erfindungsgemäßem Gemisch von Tri*iso*pentylestern der Trimellitsäure innerhalb einer kürzeren Reaktionszeit zu erhalten, als dies der Fall ist, wenn das gesamte Gemisch isomerer Pentanole vor Erreichen der Siedetemperatur im Kontakt mit der Trimellitsäure und/oder zum Trimellitsäurederivat steht.

Nach Beendigung der Veresterung oder Umesterung wird das jeweilige Reaktionsgemisch in üblicher Art und Weise aufgearbeitet.

So ist es beispielsweise möglich, den rohen Ester bei einem erhöhten Druck, welcher mindestens so groß ist wie der Dampfdruck von Wasser bei der vorherrschenden Temperatur, mit einer wässrigen Base zu behandeln. Diese Verfahrensführung ermöglicht es, gut filtrierbare Reaktionsmischungen zu erhalten.

In den Verfahren zur Herstellung erfindungsgemäßer Gemische von Tri*iso*pentylestern der Trimellitsäure werden vorzugsweise Gemische primärer Pentanole eingesetzt um Weichmacher zu erhalten, die eine gute Verarbeitbarkeit und hohe Weichmachereffizienz sowie niedrige Flüchtigkeit aufweisen.

Geeignete Gemische primärer Pentanole sind mittels Hydroformylierung von Butenen und anschließende oder gleichzeitige Hydrierung herstellbar. Es kann vorteilhaft sein, das Gemisch isomerer Butene vor der Zuführung zur Hydroformylierung zu fraktionieren und so bestimmte Butenisomere an- beziehungsweise abzureichern, um nach Hydroformylierung und Hydrierung Gemische isomerer Pentanole einer solchen Isomerenverteilung zu erhalten, die es ermöglicht, erfindungsgemäße Gemische von Tri*iso*pentylestern der Trimellitsäure herzustellen. Für eine ausführliche Darlegung zur Steuerung der Isomerenverteilung von Pentanolen sei auf die entsprechenden Passagen in der Anmeldung US 2007/0287781 A1 verwiesen.

Weitere alternative Zugangswege zu den erfindungsgemäßen Gemischen bestehen darin, beispielsweise mittels einer Hydroformylierung hergestellte Aldehyde zu fraktionieren und auf diese Weise bestimmte Aldehydisomere an- beziehungsweise abzureichern, um nach der Hydrierung Gemische isomerer Pentanole einer solchen Isomerenverteilung zu erhalten, die es ermöglicht, erfindungsgemäße Gemische von Tri*iso*pentylestern der Trimellitsäure herzustellen. Alternativ ist es zudem möglich, Gemische isomerer Pentanole zu fraktionieren und auch hier bestimmte Pentanolisomere an- beziehungsweise abzureichern.

Alternativ ist es des Weiteren möglich, ein Gemisch isomerer Pentanole durch das Mischen von isomerenreinen Pentanolen herzustellen und dieses in einer Veresterung oder Umesterung einzusetzen. Auch die Abmischung von Gemischen isomerer Pentanole, welche eventuell nicht die zur Verwirklichung der vorliegenden Erfindung benötigte Isomerenverteilung aufweisen, mit anders zusammengesetzten Gemischen isomerer Pentanole und/oder mit einem oder mehreren isomerenreinen Pentanolen ist möglich, um nach der Veresterung oder Umesterung Gemische von Tri*iso*pentylestern der Trimellitsäure mit den geforderten Eigenschaften zu erhalten.

Des Weiteren ist es möglich, die erfindungsgemäßen Gemische herzustellen, indem nicht die in einer Veresterung oder Umesterung einzusetzenden Gemische isomerer Pentanole gezielt zusammengemischt werden, sondern Isomere des Tripentylesters der Trimellitsäure gezielt abgemischt werden.

Dementsprechend ist ein Gegenstand der vorliegenden Erfindung die Verwendung von Gemischen von Tri*iso*pentylestern der Trimellitsäure umfassend isomere Pentylreste, bei welcher die Gemische von Tri*iso*pentylestern der Trimellitsäure hergestellt werden, indem

- mindestens zwei isomerenreine Tripentylester der Trimellitsäure, die sich in der Isomerie der im Ester gebundenen Pentylreste unterscheiden,
- mindestens zwei Gemische von Tri*iso*pentylestern der Trimellitsäure umfassend isomere Pentylreste, die sich in den Isomerenverteilungen der im Estergemisch gebundenen isomeren Pentylreste unterscheiden, oder
- mindestens ein isomerenreiner Tripentylester der Trimellitsäure und mindestens ein Gemisch von Tri*iso*pentylestern der Trimellitsäure umfassend isomere Pentylreste miteinander vermischt werden, so dass mindestens 20 Mol-%, dazu bevorzugt mindestens 25 Mol-%, besonders bevorzugt mindestens 50 Mol-%, weiter bevorzugt mindestens 67 Mol-%, ganz besonders bevorzugt mindestens 75 Mol-% und insbesondere mindestens 80 Mol-% der im zusammengemischten Estergemisch gebundenen isomeren Pentylreste verzweigt sind.

Gegenstand der vorliegenden Erfindung ist die Verwendung von erfindungsgemäßen Gemischen von Tri*iso*pentylestern der Trimellitsäure umfassend isomere Pentylreste als Weichmacher oder als Teil einer Weichmacherzusammensetzung, welche neben den Gemischen der Tri*iso*pentylester der Trimellitsäure mindestens eine weitere Polymer-weichmachende Verbindung enthält, für PVC oder Vinylchlorid-haltige Copolymere.

Bevorzugte Polymere sind Polyvinylchlorid oder Co-Polymere von Vinylchlorid mit Vinylacetat oder mit Butylacrylat.

Vorzugsweise werden die erfindungsgemäßen Gemische von Tri*iso*pentylestern der Trimellitsäure als Weichmacher oder als Teil einer Weichmacherzusammensetzung in Klebstoffen, Dichtungsmassen, Beschichtungsmassen, Lacken, Farben, Plastisolen, Schäumen, Kunstleder, Fußbodenbelägen (z.B. Deckschicht), Dachbahnen, Unterbodenschutz, Gewebebeschichtungen, Kabeln, Drahtisolierungen, Schläuchen, Extrusionsartikeln, Folien, im Automobilinnenbereich, in Tapeten, Tinten, Spielzeug, Kontaktfolien, Lebensmittelverpackungen oder medizinischen Artikeln, beispielsweise Schläuchen oder Blutbeuteln, verwendet.

In einer weiteren Ausführungsform werden die erfindungsgemäßen Gemische von Tri*iso*pentylestern als Lösemittel für Additive, beispielsweise als Lösemittel für Haftvermittler eingesetzt.

Wie bereits oben erwähnt, können die erfindungsgemäßen Gemische von Tri*iso*pentylestern der Trimellitsäure mit einer oder mehreren weiteren Polymer-weichmachenden Verbindungen zusammen eine Weichmacherzusammensetzung bilden. Je nach Anwendungszweck werden ein oder mehrere Polymer-weichmachende Verbindungen mit den erfindungsgemäßen Gemischen der Tri*iso*pentylester der Trimellitsäure abgemischt und so die Eigenschaften der resultierenden Weichmacherzusammensetzung gezielt eingestellt. Eine besonders bevorzugte Ausführungsform einer erfindungsgemäßen Weichmacherzusammensetzung liegt vor, wenn diese weniger als 5 Massen-% und insbesondere weniger als 0,5 Massen-% und ganz besonders weniger als 0,1 Massen-% Phthalat-haltige Verbindungen enthält. Die weiteren Polymer-weichmachenden Verbindungen werden vorzugsweise ausgewählt aus der Gruppe der Adipate, Benzoate, beispielsweise Monobenzoate oder Glycoldibenzoate, chlorierten Kohlenwasserstoffe, Citrate, Cyclohexandicarboxylate, epoxidierten Fettsäureester, epoxidierten Pflanzenöle, epoxidierten acylierten Glyceride, Furandicarboxylate, Phosphate, Phthalate (vorzugsweise in möglichst geringen Mengen), Succinate, Sulfonamide, Sulfonate, Terephthalate, Trimellitate oder oligomeren oder polymeren Ester auf Basis von Adipin-, Bernstein- oder Sebacinsäure.

Bevorzugt werden die erfindungsgemäßen Gemische innerhalb einer Weichmacherzusammensetzung umfassend erfindungsgemäße Gemische von Tri*iso*pentylestern der Trimellitsäure umfassend isomere Pentylreste sowie mindestens eine weitere Polymer-weichmachende Verbindung aus der Gruppe der Alkylbenzoate, Dialkyladipate, Glycerinester, Citronensäuretrialkylester, acylierten Citronensäuretrialkylester, Glykoldibenzoate, Trimellitate mit anderen Resten als in der vorliegenden Erfindung beschrieben, Dialkylterephthalate, Dialkylphthalate, Ester der Furandicarbonsäure, Dialkanoylester von Dianhydrohexitolen (z.B. Isosorbid), epoxidierten Fettsäurealkylester, Polymerweichmacher, beispielsweise der Polyadipate, und Dialkylester der 1,2-, 1,3- oder 1,4-Cyclohexandicarbonsäure verwendet.

Ein bevorzugter Gegenstand der vorliegenden Erfindung ist die Verwendung einer Weichmacherzusammensetzung umfassend erfindungsgemäße Gemische von Tri*iso*pentylestern der Trimellitsäure umfassend isomere Pentylreste und Di-isononylterephthalat (DINT). Entsprechende Weichmacherzusammensetzungen zeichnen sich durch eine niedrige Flüchtigkeit aus. Diese Weichmacherzusammensetzungen sind besonders für Hochtemperaturanwendungen wie Kabel vorteilhaft einsetzbar, da Produkte enthaltend entsprechende Weichmacherzusammensetzungen eine gute Hitzebeständigkeit aufweisen.

Ein weiterer bevorzugter Gegenstand der vorliegenden Erfindung ist die Verwendung einer Weichmacherzusammensetzung umfassend erfindungsgemäße Gemische von Tri*iso*pentylestern der Trimellitsäure umfassend isomere Pentylreste und 1,2- oder 1,4-Cyclohexandicarbonsäureester, insbesondere Diisononylester. Produkte, welche diese Weichmacherzusammensetzungen enthalten, zeichnen sich insbesondere dadurch aus, dass sie eine verbesserte Kälteflexibilität aufweisen. Zudem sind Plastisole enthaltend entsprechende Weichmacherzusammensetzungen aufgrund einer herabgesetzten Viskosität besser verarbeitbar.

Weiterer bevorzugter Gegenstand der vorliegenden Erfindung ist die Verwendung einer Weichmacherzusammensetzung umfassend erfindungsgemäße Gemische von Tri*iso*pentylestern der Trimellitsäure umfassend isomere Pentylreste und epoxidierte Fettsäurealkylester mit einer Kettenlänge der Alkylreste von 4 bis 6 Kohlenstoffatomen. Plastisole enthaltend entsprechende Weichmacherzusammensetzungen sind aufgrund einer herabgesetzten Viskosität und guten Gelierung gut verarbeitbar.

Ebenfalls bevorzugt ist eine Weichmacherzusammensetzung umfassend erfindungsgemäße Gemische von Tri*iso*pentylestern der Trimellitsäure umfassend isomere Pentylreste und Trialkylestern der Trimellitsäure, in denen die Alkylreste 6 und mehr, vorzugsweise 7, 8, 9, 10 oder 11 Kohlenstoffatomen enthalten. Bevorzugt sind auch Weichmacherzusammensetzungen umfassend erfindungsgemäße Gemische von Tri*iso*pentylestern der Trimellitsäure umfassend isomere Pentylreste und PolymerWeichmacher. Die in diesem Absatz genannten Ausführungsformen der erfindungsgemäßen Weichmacherzusammensetzungen beziehungsweise der daraus hergestellten Produkte weisen eine besonders geringe Flüchtigkeit auf.

Weiterer Gegenstand der vorliegenden Erfindung ist eine Kunststoffzusammensetzung enthaltend ein erfindungsgemäßes Gemisch von Tri*iso*pentylestern der Trimellitsäure und ein oder mehrere Polymere aus der Gruppe, die gebildet wird von Polyvinylchlorid und Co-Polymeren von Vinylchlorid mit Vinylacetat oder mit Butylacrylat.

Bezogen auf 100 Massenteile Polymer enthalten bevorzugte Kunststoffzusammensetzungen von 5 bis 200, bevorzugt von 10 bis 150 Massenteile an Weichmacher.

Bevorzugt ist das Polymer ein Suspensions-, Masse-, Mikrosuspensions- oder Emulsions-PVC.

In einer Ausführungsform enthält die erfindungsgemäße Kunststoffzusammensetzung neben dem erfindungsgemäßen Gemisch von Tri*iso*pentylestern der Trimellitsäure weniger als 50 Massen-%, weniger als 20 Massen-%, weniger als 10 Massen-% oder keine weiteren Polymer-weichmachenden Verbindungen, wobei die Massen-% auf der Gesamtmasse der Kunststoffzusammensetzung basieren.

Erfindungsgemäße Kunststoffzusammensetzungen enthalten vorzugsweise neben dem Polymer oder einer Mischung mehrerer Polymere und dem erfindungsgemäßen Gemisch von Tri*iso*pentylestern der Trimellitsäure sowie einem oder mehreren weiteren optionalen Polymer-weichmachenden Verbindungen ein oder mehrere Additive aus der Gruppe der Thermostabilisatoren, Füllstoffe, Pigmente, Treibmittel, Biozide, UV- und Licht-Stabilisatoren, Co-Stabilisatoren, Antioxidantien, Viskositätsregler, Entlüfter, Haftvermittler, Gleitmittel und Färbemittel.

Vorzugsweise enthalten erfindungsgemäße Kunststoffzusammensetzungen Viskositätsregler, welche vorteilhafterweise eingesetzt werden um die Viskosität von Plastisolen, welche erfindungsgemäße Gemische enthalten, herabzusetzen.

Die erfindungsgemäßen Kunststoffzusammensetzungen können in Klebstoffen, Dichtungsmassen, Beschichtungsmassen, Lacken, Farben, Plastisolen, Schäumen, Kunstleder, Fußbodenbelägen (z.B. Deckschicht), Dachbahnen, Unterbodenschutz, Gewebebeschichtungen, Kabeln, Drahtisolierungen, Schläuchen, Extrusionsartikeln, Folien, im Automobilinnenbereich, in Tapeten, Tinten, Spielzeug, Kontaktfolien, Lebensmittelverpackungen oder medizinischen Artikeln, beispielsweise Schläuchen oder Blutbeuteln, eingesetzt werden.

**Beispiele**

Beispiele 1 bis 7: Herstellung von Trimellitaten durch Veresterung von Trimellitsäureanhydrid

In eine Apparatur umfassend Rührkolben mit Rührer, Probenahmestutzen, Tropftrichter, Tauchrohr, Thermometer und Wasserabscheider mit aufgesetztem Intensivkühler, wobei dem Wasserabscheider im Falle der Herstellung von $C_4$-Trimellitat eine 20 cm Raschigringkolonne vorgeschaltet war, wurden 576,0 g (3 mol) Trimellitsäureanhydrid (Sigma Aldrich, Reinheit 97 %) und die Menge $m_1$ des Alkohols A eingefüllt. Die Apparatur wurde eine Stunde mit Stickstoff (6 l/h) über das Tauchrohr gespült, bevor 0,8 g (0,75 mmol) Tetra-n-butyltitanat (Sigma Aldrich, Reinheit > 97 %) hinzugegeben wurden. Während einer bis zur Beendigung der Reaktion andauernden Einperlung von Stickstoff (6 l/h) wurde das Gemisch unter Rühren zum Sieden erhitzt. Durch die Reaktion anfallendes Wasser wurde kontinuierlich mittels des Wasserabscheiders entfernt. Ab einer Reaktionstemperatur von 240 °C wurde die Menge $m_2$ des Alkohols A und 0,8 g (0,75 mmol) Tetra-n-butyltitanat in einer solchen Geschwindigkeit zudosiert, dass die Reaktionstemperatur nicht unter 240 °C sank. Sobald mittels des Wasserabscheiders 108 ml (6 Mol) Wasser abgetrennt worden waren, wurde anhand einer Probe der Reaktionslösung die Säurezahl in Anlehnung an die DIN EN ISO 2114 bestimmt. In Abhängigkeit der jeweils bestimmten Säurezahl wurde das Reaktionsgemisch weiter erhitzt, bis die Säurezahl des Reaktionsgemisches unterhalb von 0,1 mg KOH pro g Probe lag (Gesamtreaktionszeit t).

Das abgekühlte Reaktionsgemisch aus der Veresterung wurde in einen Rührkolben mit Rührer, Thermometer, Tauchrohr, Claisen-Brücke und Vorlagekolben umgefüllt, mindestens eine Stunde mit Stickstoff über das Tauchrohr gespült und anschließend der Druck auf ca. 1 mbar reduziert. Die Temperatur wurde langsam bis auf 160 °C erhöht und der Überschussalkohol abdestilliert. Es wurde unter Vakuum und Einleitung von Stickstoff (20 mbar) abgekühlt. Bei Erreichen einer Temperatur von 80 °C wurde der Druck mit Stickstoff ausgeglichen, das Rohprodukt mit 15 mmol vollentsalztem Wasser und 0,5 Massen-% Aktivkohle (Cabot Norit Nederland B.V., CAP Super, Menge bezogen auf das Reaktionsprodukt bei angenommener Ausbeute von 100 %) versetzt und bei 80 °C für 15 Minuten unter Stickstoffeinleitung (6 l/h) gerührt. Anschließend wurde das Reaktionsgemisch 2 Stunden unter Vakuum bei Stickstoffeinleitung (20 mbar) auf 160 °C erhitzt und letzte flüchtige Komponenten abgetrennt. Nach erneutem Abkühlen auf 80 °C wurde das Reaktionsgemisch unter Stickstoffeinperlung (6 l/h) mit 2 Gew.-% basischem Aluminiumoxid (Sigma Aldrich, Typ Brockmann 1, Menge bezogen auf das Reaktionsprodukt bei angenommener Ausbeute von 100 %) vermischt und bei 80 °C für 1 Stunde gerührt.

Das Reaktionsgemisch wurde anschließend bei 80 °C über einen Büchnertrichter mit Filterpapier und vorgepresstem Filterkuchen aus Filterhilfsmittel (Perlite Typ D14 ex Knauf) mittels Vakuum in eine Saugflasche filtriert. Das Filtrat wurde mittels GC-Analyse hinsichtlich der Reinheit und mittels NMR hinsichtlich der Zusammensetzung untersucht.

GC-Analysen:

Die GC- Analyse erfolgte mit folgenden Parametern:

Kapillarsäule: 30 m DB5; 0,25 mm ID; 0,25 $\mu$m Film
Trägergas: Helium
Säulenvordruck: 150 kPa
Cool on Column Injektion
Ofentemperaturprogramm (Dauer: 51 min): 50 °C (für 1 min), Aufheizen mit 15 °C/min auf 350 °C (Temperatur halten für 14 min)
Injektor: 50 °C
Detektor (FID): 425 °C
Injektionsvolumen: 0,3 $\mu$l

Die Identifizierung der Komponenten im Chromatogramm der Probe erfolgte mittels einer Vergleichslösung der relevanten Ester. Im Anschluss erfolgte eine Normierung der Signale im Chromatogramm der Probe auf 100 Flächen-%. Die Stoffmengenverhältnisse wurden in hinreichender Näherung aus den Flächenverhältnissen der einzelnen Signale bestimmt. Die Reinheit wurde über den Anteil der Produktsignale an den Gesamtflächen im Chromatogramm bestimmt.
Die Art und Anzahl der in den Tri*iso*pentylester enthaltenen isomeren Pentylreste kann alternativ zu der unten beschriebenen NMR-Methode durch Verseifung des Estergemisches in basischer Lösung und nachfolgender GC-Analyse des resultierenden Alkoholgemisches bestimmt werden. Hierbei ist zu beachten, dass die GC-Bedingungen (insbesondere Säulenmaterial und Säulenabmessungen sowie Temperaturprogramm) eine Auftrennung der Alkohole in die einzelnen Isomere zulassen. Die Identifizierung der Komponenten im Chromatogramm der Probe erfolgt dann mittels einer Vergleichslösung der relevanten Ester. Im Anschluss erfolgt eine Normierung der Signale im Chromatogramm der Probe auf 100 Flächen-%, so dass die Bestimmung der Stoffmengenverhältnisse in hinreichender Näherung aus den Flächenverhältnissen der einzelnen Signale möglich ist.

NMR-Analysen:

Die Ermittlung der Zusammensetzung der Tri*iso*pentylestergemische, das heißt der jeweilige Anteil der unterschiedlichen isomeren Pentylreste an der Gesamtheit aller Pentylreste, kann beispielsweise durch $^1$H-NMR und $^{13}$C-NMR Spektroskopie erfolgen. Die Ermittlung der Zusammensetzung wurde hier mit Hilfe der $^1$H-NMR-Spektroskopie an einer Lösung der Tri*iso*pentylestergemische in Deuterochloroform ($CDCl_3$) durchgeführt. Für die Aufnahme der Spektren wurden 20 mg Substanz in 0,6 ml $CDCl_3$ (enthaltend 1 Massen-% TMS) gelöst und in ein NMR-Röhrchen mit einem Durchmesser von 5 mm gefüllt. Sowohl die zu untersuchende Substanz als auch das verwendete $CDCl_3$ wurden zunächst über Molekularsieb getrocknet, um Verfälschungen der Messwerte durch eventuell vorhandenes Wasser auszuschließen. Die NMR-spektroskopischen Untersuchungen können prinzipiell mit jedem handelsüblichen NMR-Gerät durchgeführt werden. Für die vorliegenden NMR-spektroskopischen Untersuchungen wurde ein Gerät vom Typ Avance 500 der Firma Bruker eingesetzt. Die Spektren wurden bei einer Temperatur von 303 K mit einem Delay (Verzögerung) von d1 = 5 Sekunden, 32 Scans (Durchgänge), einer Pulslänge von ca. 9,5 $\mu$s (90° Anregungspuls) und einer Sweep Width (Spektrale Breite) von 10000 Hz mit einem 5 mm BBO-Probenkopf (broad band observer; Breitbandbeobachtung) aufgenommen. Die Resonanzsignale wurden gegen die chemischen Verschiebungen von Tetramethylsilan (TMS = 0 ppm) als internen Standard aufgezeichnet. Mit anderen handelsüblichen NMR-Geräten werden mit den gleichen Betriebsparametern vergleichbare Ergebnisse erhalten.
Die folgende Abbildung 1 soll lediglich der Nachvollziehbarkeit der erläuterten Bestimmungsmethode dienen. Relevant hierbei ist die zugewiesene Nummerierung der Kohlenstoffatome der Methylgruppen sowie der Methylengruppen benachbart zum Sauerstoffatom jeweils in einem *n*-Pentylrest ($C^{14}H_3$ und $C^{10}H_2$), in einem 2-Methylbutylrest ($C^{22}H_3$, $C^{23}H_3$ und $C^{19}H_2$) und in einem 3-Methylbutylrest ($C^{18}H_3$, $C^{18'}H_3$ und $C^{15}H_2$).

Abbildung 1: Nummerierung der Kohlenstoffatome in den unterschiedlichen Pentylresten

Die erhaltenen ¹H-NMR-Spektren der Gemische von Tri*iso*pentylestern der Trimellitsäure weisen im Bereich von 0,5 ppm bis zum Minimum des niedrigsten Tals im Bereich von 0,7 bis 1,2 ppm Resonanzsignale auf, die durch die Signale der Wasserstoffatome der Methylgruppe(n) der isomeren Pentylsubstituenten gebildet werden ($C^{14}H_3$, $C^{18}H_3$, $C^{18'}H_3$, $C^{22}H_3$, $C^{23}H_3$). Die Signale im Bereich der chemischen Verschiebungen von 3,60 bis 4,55 ppm können im Wesentlichen den Wasserstoffatomen der Methylengruppe, welche dem Sauerstoff des Alkoholrests benachbart ist, zugeordnet werden ($C^{10}H_2$, $C^{15}H_2$, $C^{19}H_2$). Hierbei erfahren die Protonen an C19 aufgrund des benachbarten tertiären Kohlenstoffatoms eine Hochfeldverschiebung und erscheinen zwischen 3,95 und 4,29 ppm, während die Protonen an C10 und C15 Signale bei tieferen Verschiebungen von 4,29 bis 4,55 ppm liefern.

Die Quantifizierung erfolgte durch vergleichende Bestimmung der Fläche unter den jeweiligen Resonanzsignalen, d. h. der vom Signal von der Grundlinie eingeschlossenen Fläche. Handelsübliche NMR-Software verfügt über Programmfunktionen zur Integration der Signalfläche. In der vorliegenden NMR-spektroskopischen Untersuchung wurde die Integration mit Hilfe der Software TopSpin®, Version 3.1 durchgeführt.

Zur Bestimmung des mittleren Verzweigungsgrades der isomeren Pentylreste im erfindungsgemäßen Gemisch wird zunächst der Integralwert der Signale im Bereich von 0,68 bis 1,18 ppm ($I(CH_3)$) durch den Integralwert der Signale im Bereich von 3,95 bis 4,55 ppm ($I(OCH_2)$) dividiert. Auf diese Weise erhält man ein Intensitätsverhältnis, welches das Verhältnis der Anzahl der Wasserstoffatome, die in einer Methylgruppe vorhanden sind, zur Anzahl der Wasserstoffatome, die in einer einem Sauerstoff benachbarten Methylengruppe vorhanden sind, angibt. Da pro Methylgruppe drei Wasserstoffatome und in jeder einem Sauerstoff benachbarten Methylengruppe zwei Wasserstoffatome vorhanden sind, müssen die Intensitäten jeweils durch 3 bzw. 2 geteilt werden, um das Verhältnis der Anzahl der Methylgruppen zur Anzahl der einem Sauerstoff benachbarten Methylengruppe im Pentylrest zu erhalten. Da ein linearer *n*-Pentylrest, welcher nur eine Methylgruppe und eine einem Sauerstoff benachbarte Methylengruppe aufweist, keine Verzweigung enthält und demnach einen Verzweigungsgrad von 0 aufweisen muss, muss von dem Verhältnis noch der Betrag 1 subtrahiert werden.

Der mittlere Verzweigungsgrad V kann also gemäß der Formel

$$V = 2/3 * I(CH_3)/I(OCH_2) - 1$$

aus dem gemessenen Intensitätsverhältnis berechnet werden. Hierin bedeutet V mittlerer Verzweigungsgrad, $I(CH_3)$ Flächenintegral, welches den Methylwasserstoffatomen zugeordnet ist, und $I(OCH_2)$ Flächenintegral der Methylenwasserstoffatome in Nachbarschaft zum Sauerstoff.

Im Produkt können 2-Methylbutylreste und 3-Methylbutylreste, welche jeweils einen Verzweigungsgrad von 1 aufweisen, sowie *n*-Pentylreste, welche einen Verzweigungsgrad von 0 besitzen, enthalten sein, womit der mittlere Verzweigungsgrad eines Tri*iso*pentylesters stets bei maximal 1 liegt. Aus der Abweichung des mittleren Verzweigungsrades von dem Wert 1 kann daher der Stoffmengenanteil an *n*-Pentylresten ($x_{Pentyl}$) im Molekül ermittelt werden.

$$x_{Pentyl} = 1 - V$$

Der Anteil an 2-Methylbutylresten kann mit Hilfe der Integration der Basislinien-getrennten Signale im Bereich von 3,95 bis 4,55 ppm berechnet werden. Die Trennung der Signale der Protonen an C19 ($C^{19}H_2$; Multiplett zwischen 3,95 und 4,28 ppm) von den Signalen der Protonen an C10 und C15 ($C^{10}H_2$ und $C^{15}H_2$; Multiplett zwischen 4,29 und 4,55 ppm) erfolgt auch hier im Minimum des Taleinschnittes zwischen den Signalgruppen.

Der Stoffmengenanteil an 2-Methylbutylresten ($x_{2-Methylbutyl}$) lässt sich gemäß der Formel

$$x_{2-Methylbutyl} = I(OC^{19}H_2) / I(OCH_2)$$

durch das In-Verhältnis-Setzen der Intensität der Signale für die $OC^{19}H_2$-Protonen ($I(OC^{19}H_2)$) zu der Intensität aller $OCH_2$-Protonen ($I(OCH_2)$) berechnen.

Der Stoffmengenanteil an 3-Methylbutylresten ($x_{3-Methylbutyl}$) ergibt sich somit aus der Differenz der beiden vorherigen Stoffmengenanteile zu 1.

$$x_{3-Methylbutyl} = 1 - x_{2-Methylbutyl} - x_{Pentyl}$$

Tabelle 1: Herstellung von Trimellitaten durch Veresterung von Trimellitsäureanhydrid

| Bsp. | Alkohol A [Molverhältnis] | mi(A) [g] | $m_2$(A) [g] | Reaktionszeit t [h] | Reinheit gemäß GC [%] | Zusammensetzung: Anteile der Reste im Estergemisch [Molverhältnis] |
|---|---|---|---|---|---|---|
| 1 | *n*-Butanol | 416,9 | 352,1 | 5 | 97,8 | *n*-Butyl |
| 2 | *n*-Pentanol | 497,4 | 497,4 | 9 | 99,2 | *n*-Pentyl |
| 3 | *n*-Hexanol | 574,8 | 574,8 | 2,5 | 98,0 | *n*-Hexyl |
| 4* | 2-Methylbutanol/ *n*-Pentanol 1:4 | 495,8 | 495,8 | 8,5 | 99,3 | 2-Methylbutyl / *n*-Pentyl 0,20 : 0,80 |
| 5* | 2-Methylbutanol/ *n*-Pentanol 1:1 | 396,7 | 471,0 | 8 | 98,8 | 2-Methylbutyl / *n*-Pentyl 0,50 : 0,50 |
| 6* | 2-Methylbutanol/ 3-Methylbutanol/ *n*-Pentanol 1:1:1 | 495,8 | 495,8 | 7 | 98,9 | 2-Methylbutyl / 3-Methylbutyl / *n*-Pentyl 0,33 : 0,33 : 0,34 |
| 7* | 2-Methylbutanol/ *n*-Pentanol 4:1 | 495,8 | 495,8 | 8 | 99,0 | 2-Methylbutyl / *n*-Pentyl 0,79 : 0,21 |
| erfindungsgemäß | | | | | | |

Beispiel 8: Herstellung von Dryblends, Walzfellen und Pressplatten

Die für die nachfolgenden Beispiele benötigten Prüfkörper werden durch Trockenvermischen (Dryblendherstellung), Kalandrieren (Walzen) und Pressen der folgenden Rezepturen hergestellt:

Tabelle 2: Dryblend-Rezeptur

| | phr |
|---|---|
| PVC (Solvin S271 PC; Fa. Solvay) | 100 |
| Ester oder Estergemisch aus Beispiel 1, 2, 3, 4*, 5*, 6*, 7*, Tri-2-ethylhexyltrimellitat beziehungsweise DINP | 67 |
| Co-Stabilisator - Epoxidiertes Sojabohnenöl (Drapex 39 ex Galata) | 3 |
| Thermostabilisator auf Ba/Zn Basis (Mark BZ 965 ex Galata) | 2 |

(fortgesetzt)

| | phr |
|---|---|
| Verarbeitungshilfsmittel - Fettsäuresalze (Mark CD 41-0137 ex Galata) | 0,4 |

phr: parts per hundred parts resin
Tri-2-ethylhexyltrimellitat: Trioctyltrimellitat ex Sigma Aldrich, Reinheit 99%
DINP: Vestinol 9 ex Evonik Industries, Reinheit > 99%

Mit Trockenmischungen, die als Dryblends bezeichnet werden, lassen sich zum Beispiel nach thermoplastischer Verarbeitung (z.B. Kalandrieren oder Extrudieren) Kabel- und Drahtisolierungen, Schläuche oder Fußböden und Dachbahnen herstellen.

Die Herstellung der Dryblends erfolgte in einem Brabender Planetenmischer. Ein Thermostat (Firma Lauda, Typ RC6) sorgte für die Temperierung des Mischbehälters am Planetenmischer. Ein PC zeichnete die vom Mischer gesendeten Daten auf.

Über die Software "Winmix" wurden folgende Parameter am Brabender Planetenmischer eingestellt.

| | |
|---|---|
| Drehzahlprogramm: | Aktiv |
| Profil: | Drehzahl 50 U/min; Haltezeit: 9 min; |
| | Anstiegszeit (der Drehzahl): 1 min; |
| | Drehzahl 100 U/min; Haltezeit: 20 min |
| Temperatur: | 88 °C |
| Messbereich: | 2 Nm |
| Dämpfung: | 3 |

Die Temperatur im Mischbehälter betrug nach einstündiger Temperierzeit 88 °C. Nachdem der Planetenmischer eine Eigenkalibrierung durchgeführt hatte, wurden die festen Bestandteile (PVC, Stabilisator), welche vorher auf einer Waage (Firma Mettler, Typ XS6002S) in vierfacher Menge (vierfache Menge in g bezogen auf die Tabelle 2 in phr) in einen PE-Becher einwogen wurden, dem Mischgefäß über einen Feststofftrichter und den vorhandenen Einfüllstutzen am Brabender Mischgefäß zugeführt. Das Programm wurde gestartet und die Pulvermischung wurde 10 Minuten im Mischgefäß gerührt und temperiert, ehe die flüssigen Bestandteile, welche ebenfalls in vierfacher Menge auf der Waage in einem PE-Becher ausgewogen wurden, über einen Flüssigkeitstrichter und den vorhandenen Einfüllstutzen am Brabender Mischgefäß, zugeführt wurden. Die Mischung wurde weitere 20 Minuten im Planetenmischer gerührt. Nach Beendigung des Programms wurde die fertige Trockenmischung (Dryblend) entnommen.

Aus diesen Dryblends wurden Walzfelle hergestellt. Die Herstellung der Walzfelle erfolgte auf einem Kalander W150 AP der Firma Collin. Der Kalander der Firma Collin besitzt einen automatischen Probenumleger und wird über einen zusätzlichen Ölthermostat (Firma Single, Typ STO 1-6-12-DM) temperiert. Die Steuerung erfolgte über Software der Fa. Collin.

Es wurde ein fünfstufiges Programm zur Herstellung des Walzfelles verwendet:

| Stufe | Bezeichnung | Temp. [°C] | Dauer [s] | Spaltbreite [mm] | Drehzahl [Upm] |
|---|---|---|---|---|---|
| 1 | Plastifizierung des Dryblends | 165 | 60 | 0,2 | 5 |
| 2 | Vergrößerung des Spalts | 165 | 30 | 0,5 | 20 |
| 3 | Aktivierung des Probenumlegers | 165 | 170 | 0,5 | 20 |
| 4 | Walzfelloptimierung | 165 | 30 | 0,5 | 25 |
| 5 | Walzfellabnahme | 165 | 60 | 0,5 | 7 |

Nach dem Erreichen der Walzentemperatur wurde der Walzenspalt kalibriert. Zum Start der Messung wurde der Walzenspalt auf 0,2 mm eingestellt. Jeweils 160 g eines Dryblends wurden eingewogen und bei stehenden Walzen in den Walzenspalt gegeben. Das Programm wurde gestartet. Die Walzen starteten mit einer Umdrehungszahl von 5 U/min und einer Friktion von 20 %. Nach ca. 1 min war die Plastifizierung zum größten Teil abgeschlossen und der Walzenspalt wurde auf 0,5 mm vergrößert. Es erfolgte eine 6-malige Homogenisierung mittels automatischer Umlegeeinheit am Kalander. Nach etwa 6 min wurde das Walzfell von der Walze entfernt und abgekühlt.

Die Pressplatten wurden mit einer Laborpresse der Firma Collin hergestellt. Die vorgefertigten Walzfelle (siehe oben) wurden zur Herstellung der Pressplatten verwendet. Die Seitenränder der Walzfelle wurden mit Hilfe einer Schneidemaschine entfernt, das Walzfell wurde anschließend in ca. 14,5 x 14,5 cm große Stücke geschnitten. Für 1 mm dicke Pressplatten wurden je 2 Walzfellstücke übereinander in den 15 x 15 cm großen Pressrahmen aus Edelstahl gelegt.

Es wurde dreistufiges Programm zur Herstellung der Pressplatten verwendet:

| Stufe | Bezeichnung | Temp. [°C] | Druck [bar] | Dauer [s] |
|-------|-------------|-----------|-------------|-----------|
| 1 | Vorpressen | 175 | 5 | 60 |
| 2 | Pressen | 175 | 200 | 120 |
| 3 | Abkühlen | 40 | 200 | 270 |

Die überschüssige Presslippe wurde nach Herstellung der Pressplatten entfernt.

Beispiel 9: Weichmacheraufnahme im Dryblend

Während der Herstellung jedes Dryblends wurde die Geschwindigkeit der Weichmacheraufnahme bestimmt, welche als Maß für die Verarbeitbarkeit dient. Eine niedrige Weichmacheraufnahmezeit resultiert in der Praxis in niedrigen Verarbeitungstemperaturen oder einem erhöhten Produktdurchsatz auf einer bestehenden Anlage.

Der Grad der Weichmacheraufnahme wurde über das zeitabhängige Drehmoment des Mischers bei der Dryblend-Herstellung bestimmt.

Dazu wurde mittels der im Beispiel 8 für die Dryblend-Herstellung beschriebenen Hard- und Software das Drehmoment des Mischers gegen die Zeit aufgetragen. Das Drehmoment stieg ab der Zugabe des Weichmachers beziehungsweise der Weichmacherzusammensetzung auf einen Maximalwert an und fiel ab diesem wieder ab, um schließlich ab dem so genannten Trockenpunkt einen konstanten Wert zu erreichen oder nur noch minimal abzufallen.

Die Zeitspanne zwischen der Zugabe des Weichmachers beziehungsweise der Weichmacherzusammensetzung und dem Trockenpunkt wird als Zeit t zur Weichmacheraufnahme bezeichnet.

Tabelle 3: Weichmacheraufnahme Dryblend

| | Rest(e) im Trialkyltrimellitat(-gemisch) | Zeit t [min] zur Weichmacheraufnahme |
|---|---|---|
| Ester aus Beispiel 3 | *n*-Hexyl | 4,9 |
| Trioctyltrimellitat ex Sigma Aldrich, Reinheit 99% | 2-Ethylhexyl | 9,0 |
| Estergemisch aus Beispiel 4* | 2-Methylbutyl / *n*-Pentyl Molverhältnis 0,20 : 0,80 | 4,1 |
| Estergemisch aus Beispiel 5* | 2-Methylbutyl / *n*-Pentyl Molverhältnis 0,50 : 0,50 | 4,4 |
| Estergemisch aus Beispiel 6* | 2-Methylbutyl / 3-Methylbutyl / *n*-Pentyl Molverhältnis 0,33 : 0,33 : 0,34 | 4,5 |
| Estergemisch aus Beispiel 7* | 2-Methylbutyl / *n*-Pentyl Molverhältnis 0,79 : 0,21 | 4,6 |
| Vestinol 9 ex Evonik Industries, Reinheit > 99% | Vergleichssubstanz: Di*iso*nonylphthalat (DINP) | 5,4 |
| * erfindungsgemäß | | |

Im Falle der erfindungsgemäßen Gemische von Tri*iso*pentylestern der Trimellitsäure aus den Beispielen 4*, 5*, 6* und 7* wurden innerhalb einer kürzeren Zeit homogene Dryblends gebildet als im Falle von Di*iso*nonylphthalat, dem Tri-*n*-hexylester der Trimellitsäure (aus Beispiel 3) und dem Tri-2-ethylhexylester der Trimellitsäure. Dies lässt direkt darauf schließen, dass erfindungsgemäße Gemische eine bessere Verarbeitbarkeit mit PVC aufweisen als DINP, als der Tri-*n*-hexylester der Trimellitsäure und als der Tri-2-ethylhexylester der Trimellitsäure. Zudem kann den Werten in Tabelle 3 entnommen werden, dass in erfindungsgemäßen Gemischen mit dem Anteil der linearen Pentylreste an den im Estergemisch gebundenen Pentylresten die Verarbeitbarkeit mit PVC steigt.

Der Tri-2-ethylhexylester der Trimellitsäure weist eine besonders hohe Weichmacheraufnahmezeit auf und ist deshalb als Weichmacher für zahlreiche PVC-Anwendungen nicht geeignet, weshalb seine Migrationseigenschaften im Folgenden nicht bestimmt wurden.

Beispiel 10: Bestimmung der Migrationseigenschaften

Der Test zur Bestimmung der Migrationseigenschaften eines Prüfkörpers enthaltend einen Weichmacher oder eine Weichmacherzusammensetzung, erlaubt es, Rückschlüsse darauf zu ziehen, wie sich Weichmacher-haltige Zubereitungen in der Anwendung bei direktem Kontakt mit anderen Materialien - wie beispielsweise bei Kontakt zwischen einer weichgemachten PVC-Schicht mit einer Hart-PVC-Schicht - verhalten (so genannte "MultiLayer Systeme). Geht beispielsweise innerhalb eines Artikels von einem weichgemachten Polymer-haltigen Bauteil Weichmacher zu einem nicht-weichgemachten Polymer-haltigen Bauteil über, kann dies zur unerwünschten Versprödung des weichgemachten Bauteils und Aufweichung des nicht-weichgemachten Bauteils führen, was zu Undichtigkeiten, nachlassender Stabilität und verringerter Gebrauchszeit des Artikels führen kann. Dementsprechend ist eine geringe Migrationsneigung eine wichtige Eigenschaft für einen in zahlreichen Anwendungen einsetzbaren Weichmacher oder eine dementsprechende Komponente einer Weichmacherzusammensetzung.

Die Migrationsneigung der erfindungsgemäßen Gemische von Tri*iso*pentylestern der Trimellitsäure und der Vergleichssubstanzen DINP, Tri-*n*-butyltrimellitat, Tri-*n*-pentyltrimellitat und Tri-*n*-hexyltrimellitat wurde wie folgt bestimmt:

Die in Beispiel 8 hergestellten, 1 mm starken Pressplatten wurden in Prüflinge von 100 mm · 100 mm geschnitten und für 24 Stunden bei 23 °C gelagert, bevor ihre Masse ermittelt wurde.

In Anlehnung an die Vorschrift DIN EN ISO 177 (veröffentlicht 1999) wurden die Prüflinge zwischen zwei Kontaktschichten (Beispiel 10.1: hochschlagfestes Polystyrol (HIPS) (Hersteller VINK Kunststoffe; 100 x 100 x 2 mm Platten); Beispiel 10.2: nichtweichgemachtes PVC (rPVC) (Hersteller VINK Kunststoffe; 100 x 100 x 2 mm Platten)), welche bis auf eine Stärke von 2 mm dieselben Dimensionen wie die Prüflinge aufwiesen, platziert und jeweils zwei der auf diese Weise entstehenden Schichtkonstruktionen übereinander gestapelt. Diese Stapel umfassend jeweils Prüflinge desselben Weichmachers wurden mit einem 2 kg Gewicht beschwert für 28 Tage in einem Ofen (70 (+/- 1) °C) gelagert.

Nach 14 Tagen und nach 28 Tagen wurde der Gewichtsverlust an jedem Prüfling bestimmt (Prozent Gewichtsverlust basierend auf dem Originalgewicht). Nach der ersten Messung wurden die Stapel wieder wie zuvor zusammengesetzt.

Die Tabelle 4 zeigt die gemittelten Werte des Gewichtsverlustes der Prüfkörper.

Tabelle 4: Migration in hochschlagfestes Polystyrol (HIPS) nach 14 beziehungsweise 28 Tagen

| Prüfling enthält ... | Rest(e) im Trialkyltrimellitat (-gemisch) | Gewichtsdifferenz nach 14 Tagen in % | Gewichtsdifferenz nach 28 Tagen in % |
|---|---|---|---|
| Ester aus Beispiel 1 | *n*-Butyl | 5,8 | 8,0 |
| Ester aus Beispiel 2 | *n*-Pentyl | 5,1 | 6,9 |
| Ester aus Beispiel 3 | *n*-Hexyl | 3,7 | 5,6 |
| Estergemisch aus Beispiel 4* | 2-Methylbutyl / *n*-Pentyl Molverhältnis 0,20 : 0,80 | 3,6 | 5,5 |
| Estergemisch aus Beispiel 5* | 2-Methylbutyl / *n*-Pentyl Molverhältnis 0,50 : 0,50 | 2,0 | 3,2 |
| Estergemisch aus Beispiel 6* | 2-Methylbutyl / 3-Methylbutyl / *n*-Pentyl Molverhältnis 0,33 : 0,33 : 0,34 | 2,5 | 3,7 |
| Estergemisch aus Beispiel 7* | 2-Methylbutyl / *n*-Pentyl Molverhältnis 0,79 : 0,21 | 1,6 | 2,7 |
| Vestinol 9 ex Evonik Industries, Reinheit > 99% | Vergleichssubstanz: Di*iso*nonylphthalat (DINP) | 11,2 | 14,6 |
| * erfindungsgemäß | | | |

Tabelle 5: Migration in Hart-PVC (rPVC) nach 14 beziehungsweise 28 Tagen

| Prüfling enthält ... | Rest(e) im Trialkyltrimellitat (-gemisch) | Gewichtsdifferenz nach 14 Tagen in % | Gewichtsdifferenz nach 28 Tagen in % |
|---|---|---|---|
| Ester aus Beispiel 1 | *n*-Butyl | 9,3 | 11,7 |

(fortgesetzt)

| Prüfling enthält ... | Rest(e) im Trialkyltrimellitat (-gemisch) | Gewichtsdifferenz nach 14 Tagen in % | Gewichtsdifferenz nach 28 Tagen in % |
|---|---|---|---|
| Ester aus Beispiel 2 | *n*-Pentyl | 9,0 | 11,3 |
| Ester aus Beispiel 3 | *n*-Hexyl | 8,0 | 10,1 |
| Estergemisch aus Beispiel 4* | 2-Methylbutyl / *n*-Pentyl Molverhältnis 0,20 : 0,80 | 6,2 | 8,3 |
| Estergemisch aus Beispiel 5* | 2-Methylbutyl / *n*-Pentyl Molverhältnis 0,50 : 0,50 | 5,9 | 7,9 |
| Estergemisch aus Beispiel 6* | 2-Methylbutyl / 3-Methylbutyl / *n*-Pentyl Molverhältnis 0,33 : 0,33 : 0,34 | 7,4 | 9,4 |
| Estergemisch aus Beispiel 7* | 2-Methylbutyl / *n*-Pentyl Molverhältnis 0,79 : 0,21 | 5,6 | 7,5 |
| Vestinol 9 ex Evonik Industries, Reinheit > 99% | Vergleichssubstanz: Di*iso*nonylphthalat (DINP) | 7,7 | 9,7 |
| * erfindungsgemäß | | | |

Aus den Tabellen 4 und 5 ist ersichtlich, dass die erfindungsgemäßen Gemische von Tri*iso*pentylestern der Trimellitsäure durchweg eine niedrigere Migrationsneigung aufweisen als DINP sowie die *n*-Butyl-, *n*-Pentyl- und *n*-Hexyltriester der Trimellitsäure. Zudem kann einem Vergleich der Messwerte für die Prüflinge enthaltend die erfinderischen Estergemische aus den Beispielen 5, 6 und 8 entnommen werden, dass die Migrationsneigung der erfindungsgemäßen Gemische mit steigendem Anteil der verzweigten Pentylreste an den im Estergemisch gebundenen Pentylresten abnimmt. Wie der Vergleich der Messwerte der Prüflinge enthaltend erfindungsgemäße Estergemische aus den Beispielen 5 bis 7 zeigt, wirkt sich ein hoher Anteil an 2-Methylbutylresten an den im Estergemisch gebundenen verzweigten isomeren Pentylreste positiv auf eine geringe Migrationsneigung aus.

**Patentansprüche**

1. Verwendung von Gemischen von Tri*iso*pentylestern der Trimellitsäure umfassend isomere Pentylreste, wobei mindestens 20 Mol-% der im Estergemisch gebundenen isomeren Pentylreste verzweigt sind und gleichzeitig mehr als 10 Mol-% der im Estergemisch gebundenen isomeren Pentylreste linear, sind als Weichmacher oder als Teil einer Weichmacherzusammensetzung, welche neben den Gemischen der Tri*iso*pentylester der Trimellitsäure mindestens eine weitere Polymer-weichmachende Verbindung enthält, für PVC oder Vinylchlorid-haltige Copolymere.

2. Verwendung gemäß Anspruch 1, **dadurch gekennzeichnet, dass** mindestens 25 Mol-% der im Estergemisch gebundenen isomeren Pentylreste verzweigt sind.

3. Verwendung gemäß einem der Ansprüche 1 oder 2, **dadurch gekennzeichnet, dass** mindestens 50 Mol-%, vorzugsweise mindestens 80 Mol-% der im Estergemisch gebundenen verzweigten isomeren Pentylreste 2-Methylbutylreste sind.

4. Verwendung gemäß einem der Ansprüche 1 bis 3, **dadurch gekennzeichnet, dass** mehr als 20 Mol-% der im Estergemisch gebundenen isomeren Pentylreste linear sind.

5. Verwendung gemäß einem der Ansprüche 1 bis 4, **dadurch gekennzeichnet, dass** die Gemische von Tri*iso*pentylestern der Trimellitsäure hergestellt werden, indem Trimellitsäure und/oder ein Trimellitsäurederivat mit einem Gemisch isomerer Pentanole umgesetzt wird/werden.

6. Verwendung gemäß Anspruch 5, **dadurch gekennzeichnet, dass** ein oder mehrere Trimellitsäuretrialkylester, in dem/denen die Alkylreste der Esterfunktionen jeweils weniger als 4 Kohlenstoffatome umfassen, mit einem Gemisch isomerer Pentanole umgeestert wird/werden.

**7.** Verwendung gemäß einem der Ansprüche 5 oder 6, **dadurch gekennzeichnet, dass** bei der Reaktion das Reaktionsgemisch zum Sieden erhitzt wird und mindestens 0,2 Mol-Äquivalent der Menge an Gemisch isomerer Pentanole, welche zur Einführung aller im Estergemisch gebundenen isomeren Pentylreste notwendig ist, dem die Trimellitsäure und/oder das Trimellitsäurederivat enthaltenden Reaktionsgemisch erst nach Erreichen der Siedetemperatur hinzugegeben werden.

**8.** Verwendung gemäß einem der Ansprüche 1 bis 4, **dadurch gekennzeichnet, dass** die Gemische von Tri*iso*pentylestern der Trimellitsäure hergestellt werden, indem

- mindestens zwei isomerenreine Tripentylester der Trimellitsäure, die sich in der Isomerie der im Ester gebundenen Pentylreste unterscheiden,
- mindestens zwei Gemische von Tri*iso*pentylestern der Trimellitsäure umfassend isomere Pentylreste, die sich in der Isomerenverteilungen der im Estergemisch gebundenen isomeren Pentylreste unterscheiden, oder
- mindestens ein isomerenreiner Tripentylester der Trimellitsäure und mindestens ein Gemisch von Tri*iso*pentylestern der Trimellitsäure umfassend isomere Pentylreste
miteinander vermischt werden.

**9.** Verwendung gemäß Anspruch 1, **dadurch gekennzeichnet, dass** das Gemisch von Tri*iso*pentylestern der Trimellitsäure umfassend isomere Pentylreste als Weichmacher oder als Teil einer Weichmacherzusammensetzung in Klebstoffen, Dichtungsmassen, Beschichtungsmassen, Lacken, Farben, Plastisolen, Schäumen, Kunstleder, Fußbodenbelägen (z.B. Deckschicht), Dachbahnen, Unterbodenschutz, Gewebebeschichtungen, Kabeln, Drahtisolierungen, Schläuchen, Extrusionsartikeln, Folien, im Automobilinnenbereich, in Tapeten, Spielzeug, Kontaktfolien, Lebensmittelverpackungen oder medizinischen Artikeln, beispielsweise Schläuchen oder Blutbeuteln, eingesetzt wird.

**10.** Kunststoffzusammensetzung enthaltend ein Gemisch von Tri*iso*pentylestern der Trimellitsäure umfassend isomere Pentylreste, wobei mindestens 20 Mol-% der im Estergemisch gebundenen isomeren Pentylreste verzweigt sind und gleichzeitig mehr als 10 Mol-% der im Estergemisch gebundenen isomeren Pentylreste linear sind, und ein oder mehrere Polymere aus der Gruppe, die gebildet wird von Polyvinylchlorid und Co-Polymeren von Vinylchlorid mit Vinylacetat oder mit Butylacrylat.

**Claims**

**1.** Use of mixtures of triisopentyl esters of trimellitic acid comprising isomeric pentyl radicals, wherein at least 20 mol% of the isomeric pentyl radicals incorporated in the ester mixture are branched, and at the same time more than 10 mol% of the isomeric pentyl radicals incorporated in the ester mixture are linear, as plasticizer, or as part of a plasticizer composition comprising at least one further polymer-plasticizing compound in addition to the mixtures of triisopentyl esters of trimellitic acid, for PVC or vinyl chloride-containing copolymers.

**2.** Use according to Claim 1, **characterized in that** at least 25 mol% of the isomeric pentyl radicals incorporated in the ester mixture are branched.

**3.** Use according to either of Claims 1 and 2, **characterized in that** at least 50 mol%, preferably at least 80 mol%, of the branched isomeric pentyl radicals incorporated in the ester mixture are 2-methylbutyl radicals.

**4.** Use according to any of Claims 1 to 3, **characterized in that** more than 20 mol% of the isomeric pentyl radicals incorporated in the ester mixture are linear.

**5.** Use according to any of Claims 1 to 4, **characterized in that** the mixtures of triisopentyl esters of trimellitic acid are prepared by reacting trimellitic acid and/or a trimellitic acid derivative with a mixture of isomeric pentanols.

**6.** Use according to Claim 5, **characterized in that** one or more trialkyl trimellitates in which the alkyl radicals of the ester functions each comprise fewer than 4 carbon atoms is/are transesterified with a mixture of isomeric pentanols.

**7.** Use according to either of Claims 5 and 6, **characterized in that**, in the reaction, the reaction mixture is heated to boiling and at least 0.2 molar equivalent of the amount of mixture of isomeric pentanols needed to introduce all the isomeric pentyl radicals incorporated in the ester mixture is added to the reaction mixture containing the trimellitic acid and/or the trimellitic acid derivative only after attainment of the boiling temperature.

8. Use according to any of Claims 1 to 4, **characterized in that** the mixture of triisopentyl esters of trimellitic acid are prepared by mixing together

   • at least two isomerically pure tripentyl esters of trimellitic acid which differ in terms of the isomerism of the pentyl radicals incorporated in the ester,
   • at least two mixtures of triisopentyl esters of trimellitic acid comprising isomeric pentyl radicals which differ in the isomer distributions of the isomeric pentyl radicals incorporated in the ester mixture, or
   • at least one isomerically pure tripentyl ester of trimellitic acid and at least one mixture of triisopentyl esters of trimellitic acid comprising isomeric pentyl radicals.

9. Use according to Claim 1, **characterized in that** the mixture of triisopentyl esters of trimellitic acid comprising isomeric pentyl radicals is used as plasticizer or as part of a plasticizer composition in adhesives, sealing compounds, coating materials, paints, inks, plastisols, foams, synthetic leather, floorcoverings (e.g. top layer), roofing membranes, underbody protection, fabric coatings, cables, wire insulation, hoses, extruded articles, films, in the automotive interior sector, in wallcoverings, toys, contact sheets, food packaging or medical articles, for example tubes or blood bags.

10. Polymer composition comprising a mixture of triisopentyl esters of trimellitic acid comprising isomeric pentyl radicals, wherein at least 20 mol% of the isomeric pentyl radicals incorporated in the ester mixture are branched and at the same time more than 10 mol% of the isomeric pentyl radicals incorporated in the ester mixture are linear, and one or more polymers from the group formed by polyvinyl chloride and copolymers of vinyl chloride with vinyl acetate or with butyl acrylate.

## Revendications

1. Utilisation de mélanges d'esters triisopentyliques de l'acide trimellitique comprenant des radicaux pentyle isomères, dans lesquels au moins 20 % en moles des radicaux pentyle isomères reliés dans le mélange d'esters sont ramifiés et simultanément plus de 10 % en moles des radicaux pentyle isomères reliés dans le mélange d'esters sont linéaires, en tant que plastifiant ou en tant que partie d'une composition de plastifiant, qui contient, outre les mélanges d'esters triisopentyliques de l'acide trimellitique, au moins un composé supplémentaire plastifiant les polymères, pour le PVC ou les copolymères contenant du chlorure de vinyle.

2. Utilisation selon la revendication 1, **caractérisée en ce qu'**au moins 25 % en moles des radicaux pentyle isomères reliés dans le mélange d'esters sont ramifiés.

3. Utilisation selon l'une quelconque des revendications 1 ou 2, **caractérisée en ce qu'**au moins 50 % en moles, de préférence au moins 80 % en moles, des radicaux pentyle isomères ramifiés reliés dans le mélange d'esters sont des radicaux 2-méthylbutyle.

4. Utilisation selon l'une quelconque des revendications 1 à 3, **caractérisée en ce que** plus de 20 % en moles des radicaux pentyle isomères reliés dans le mélange d'esters sont linéaires.

5. Utilisation selon l'une quelconque des revendications 1 à 4, **caractérisée en ce que** les mélanges d'esters triisopentyliques de l'acide trimellitique sont fabriqués par mise en réaction d'acide trimellitique et/ou d'un dérivé de l'acide trimellitique avec un mélange de pentanols isomères.

6. Utilisation selon la revendication 5, **caractérisée en ce qu'**un ou plusieurs esters trialkyliques de l'acide trimellitique dans lequel ou dans lesquels les radicaux alkyle des fonctions ester comprennent chacun moins de 4 atomes de carbone sont transestérifiés avec un mélange de pentanols isomères.

7. Utilisation selon l'une quelconque des revendications 5 ou 6, **caractérisée en ce que** le mélange réactionnel est porté à ébullition lors de la réaction, et au moins 0,2 équivalent en moles de la quantité de mélange de pentanols isomères qui est nécessaire pour l'insertion de tous les radicaux pentyle isomères reliés dans le mélange d'esters n'est ajoutée au mélange réactionnel contenant l'acide trimellitique et/ou le dérivé de l'acide trimellitique qu'une fois la température d'ébullition atteinte.

8. Utilisation selon l'une quelconque des revendications 1 à 4, **caractérisée en ce que** les mélanges d'esters triisopentyliques de l'acide trimellitique sont fabriqués par mélange les uns avec les autres de :

   - au moins deux esters tripentyliques de l'acide trimellitique isomériquement purs, qui diffèrent au niveau de l'isomérie des radicaux pentyle reliés dans l'ester,
   - au moins deux mélanges d'esters triisopentyliques de l'acide trimellitique comprenant des radicaux pentyle isomères, qui diffèrent au niveau des distributions isomériques des radicaux pentyle isomères reliés dans le mélange d'esters, ou
   - au moins un ester tripentylique de l'acide trimellitique isomériquement pur et au moins un mélange d'esters triisopentyliques de l'acide trimellitique comprenant des radicaux pentyle isomères.

9. Utilisation selon la revendication 1, **caractérisée en ce que** le mélange d'esters triisopentyliques de l'acide trimellitique comprenant des radicaux pentyle isomères est utilisé en tant que plastifiant ou en tant que partie d'une composition de plastifiant dans des adhésifs, des matériaux d'étanchéité, des matériaux de revêtement, des vernis, de peintures, des plastisols, des mousses, des cuirs artificiels, des revêtements de sols (p. ex. couche de recouvrement), des bandes de toiture, des protections de bas de caisse, des revêtements de tissus, des câbles, des isolations de fils, des tuyaux, des articles extrudés, des films, dans le domaine intérieur d'automobiles, dans des papiers peints, des jouets, des films de contact, des emballages de produits alimentaires ou des articles médicaux, par exemple des tuyaux ou des poches de sang.

10. Composition de matière plastique contenant un mélange d'esters triisopentyliques de l'acide trimellitique comprenant des radicaux pentyle isomères, dans lequel au moins 20 % en moles des radicaux pentyle isomères reliés dans le mélange d'esters sont ramifiés et simultanément plus de 10 % en moles des radicaux pentyle isomères reliés dans le mélange d'esters sont linéaires, et un ou plusieurs polymères du groupe qui est formé par le polychlorure de vinyle et les copolymères de chlorure de vinyle avec de l'acétate d'éthyle ou avec de l'acrylate de butyle.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 354700 B1
- DE 2348511 C3
- WO 9107459 A1
- EP 479260 B1
- JP 2005230058 A
- US 20070287781 A1

**In der Beschreibung aufgeführte Nicht-Patentliteratur**

- *Vinyl Plasticizers form Trimellitic Anhydride'' (Soc. Plastics Engrs. Techn. Papers.,* 1962, vol. 8 (22), 1, , 1-9
- **VON A.S. WILSON.** Plasticizers - Principles and Practice. The Institute of Materials, 1995, 166-170
- **DRECHSLER, GERHARD et al.** Trimellic and isophthalic acids and their esters. *Journal für praktische Chemie,* vol. 27, 231-238